# EUROPEAN PATENT SPECIFICATION

(11) **EP 1 193 265 B1**
(45) Date of publication and mention of the grant of the patent: **29.11.2006**
(21) Application number: 01123289.9
(22) Date of filing: 24.01.1997
(51) Int. Cl.: C07D 401/04

(54) **A process for the preparation of 4-¬2-(aryl or heterocyclo)-1H-imidazol-1-yl|benzenesulfonamides**
Ein Verfahren zur Herstellung von 4-¬2-(Aryl oder Heterocyclo)-1H-imidazol-1-yl|benzolsulfonamiden
Un procédé de préparation de 4-¬2-(aryl ou hétérocyclo)-1H-imidazol-1-yl|benzènesulfonamides

(30) Priority: 26.01.1996 US 592167
(43) Date of publication of application: 03.04.2002
(62) Divisional of application: 97901952.8
(73) Proprietor: G.D. Searle LLC, St. Louis, Missouri 63141 (US)
(72) Inventor: Khanna, Ish K., Libertyville, IL 60048 (US); Weier, Richard M., Lake Bluff, Illinois 60044 (US); Collins, Paul W., Deerfield, Illinois 60015 (US); Yu, Yi, Skokie, Illinois 60202 (US); Xu, Xiangdong, Gurnee, Illinois 60031 (US); Partis, Richard A., Evanston, Illinois 60201 (US); Koszyk, Francis J., Prospect Heights, Illinois 60070 (US); Huff, Renee M., Park Ridge, Illinois 60068 (US)
(74) Representative: Albrecht, Thomas

(56) References cited:
- WO-A-96/03388
- HOUBEN-WEYL: METHODEN DER ORGANISCHEN CHEMIE: BAND E8C : "Hetarene III / Teil 3" 1994 , GEORG THIEME VERLAG , STUTTGART, DE XP002189377 * page 26 - page 30 *
- GAUTIER J-A ET AL: "Préparation d'amidines à partir de nitriles et d'amines aromatiques en solvant ammoniac liquide" BULLETIN DE LA SOCIETE CHIMIQUE DE FRANCE., no. 1, 1970, pages 200-207, XP002170423 ISSN: 0037-8968

## Description

### Related Case

This is a divisional application of PCT/US97/00300 which is a continuation-in-part of International Application PCT/US95/09506, with an international filing date of July 27, 1995, which is a continuation-in-part of Patent Application 08/464,154, with a filing date of June 5, 1995, which is a continuation-in-part of Patent Application 08/282,395, with a filing date of July 28, 1994.

### FIELD OF THE INVENTION

This invention is in the field of the preparation of antiinflammatory pharmaceutical agents and specifically relates to the preparation of compounds for treating inflammation and inflammation-associated disorders, such as arthritis.

### BACKGROUND OF THE INVENTION

Prostaglandins play a major role in the inflammation process and the inhibition of prostaglandin production, especially production of PGG₂, PGH₂ and PGE₂, has been a common target of antiinflammatory drug discovery. However, common non-steroidal antiinflammatory drugs (NSAIDs) that are active in reducing the prostaglandin-induced pain and swelling associated with the inflammation process are also active in affecting other prostaglandin-regulated processes not associated with the inflammation process. Thus, use of high doses of most common NSAIDs can produce severe side effects, including life threatening ulcers, that limit their therapeutic potential. An alternative to NSAIDs is the use of corticosteroids, which have even more drastic side effects, especially when long term therapy is involved.

Previous NSAIDs have been found to prevent the production of prostaglandins by inhibiting enzymes in the human arachidonic acid/prostaglandin pathway, including the enzyme cyclooxygenase (COX). The recent discovery of an inducible enzyme associated with inflammation (named "cyclooxygenase-2 (COX-2)" or "prostaglandin G/H synthase II") provides a viable target of inhibition which more effectively reduces inflammation and produces fewer and less drastic side effects.

The references below that disclose antiinflammatory activity, show continuing efforts to find a safe and effective antiinflammatory agent. The novel imidazoles disclosed herein are such safe and also effective antiinflammatory agents furthering such efforts. The invention compounds are found to show usefulness *in vivo* as antiinflammatory agents with minimal side effects. The substituted imidazoles disclosed herein preferably selectively inhibit cyclooxygenase-2 over cyclooxygenase-1.

Diaryl oxazoles have been described in WO patent publication WO94/27980 as having antiinflammatory activity. Substituted 4,5-diarylimidazoles have been described in WO95/00501 and in copending U.S. application 08/281,903.

2-Alkylimidazoles have been described as having angiotensin II activity. For example, see U.S. Patent No. 5,185,351 and WO 91/00277.

U.S. Patent No. 5,207,820 to Wriede et al. describes 1-arylimidazole carboxylic esters as herbicide safeners. Specifically, ethyl [1-[2,6-dinitro-4-(methylsulfonyl)phenyl]-2-methyl-1H-imidazol-3-yl]carboxylate is described.

WO 93/14082, published July 22, 1993, describes 1-pyridyl-2-phenyl-imidazole derivatives for the treatment of interleukin-1 mediated diseases. 1-(4-Pyridyl)-2-(4-fluorophenyl)-4-methylimidazole is described. WO 95/02591, published January 26, 1995, describe tri-substituted imidazoles for the treatment of cytokine mediated diseases.

U.S. Patent No. 3,487,087, to Sarett et al., describes a method of nitration of imidazoles and specifically 1-methyl-2-[4-(methylsulfonyl)phenyl]-5-nitroimidazole.

U.S. Patent No. 5,112,532, to Ninomiya et al., describes imidazoles as an organic non-linear optical material. Specifically, 4-(4-hydroxyphenyl)-2-[2-formyl-4-(methylsulfonyl)phenyl]imidazole is described.

U.S. Patent Nos. 3,682,949 and 3,719,759, to Sarett et al., describe 2-aryl-nitroimidazoles as agents for the treatment of parasites and bacteria. Specifically, 1-(2-hydroxyethyl)-2-(4-sulfonamidophenyl)-5-nitroimidazole is described.

U.S. Patent No. 4,822,805, to Takasugi et al., describes pyridylimidazoles as antiinflammatory agents. Specifically, 2-[2-methoxy-4-(methylsulfonyl)phenyl]-4-methyl-5-(3-pyridyl)imidazole is described.

The invention's imidazolyl compounds are found to show usefulness in vivo as antiinflammatory agents with minimal side effects.

### DESCRIPTION OF THE INVENTION

The present invention relates to a process of making a compound of the formula 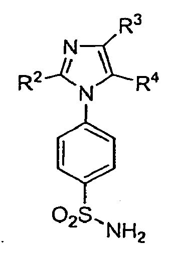 or a pharmaceutically-acceptable salt thereof, said method comprising
a) protecting the sulfonamide group of 4-nitrobenzenesulfonamide with acetonylacetone and toluenesulfonic acid to form a sulfonyl-pyrrole of the formula 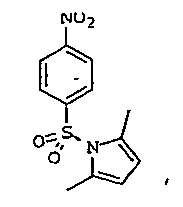
b) reducing the nitro group to form a compound of the formula 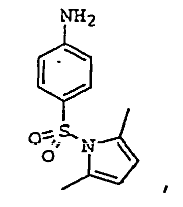
c) treating the amine with a nitrile in the presence of a base to form an amidine of the formula 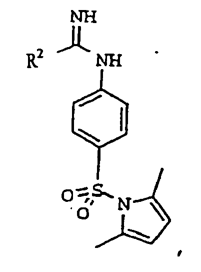
d) treating the amidine with a compound of the formula 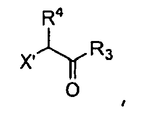 in the presence of a base to form an imidazolyl derivative of the formula 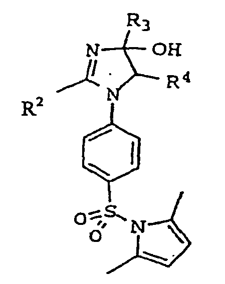
e) dehydrating the imidazolyl derivative to form a compound of the formula 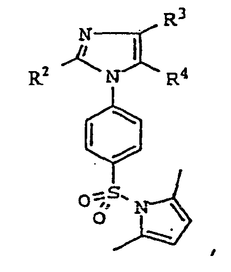
f) deprotecting the sulfonyl-pyrrole to form a compound of the formula 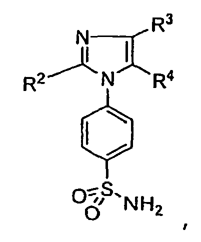 wherein
   R² is selected from imidazolyl, thienyl, thiazolyl, pyrrolyl, oxazolyl, isoxaozolyl, triazolyl, pyrimidinyl, isoquinolyl, quinolinyl, indolyl, benzimidazolyl, pyrazolyl and pyridyl, wherein R² is optionally substituted at a substitutable position with one or more radicals independently selected from methylthio, methyl, ethyl, isopropyl, *tert*-butyl, isobutyl, pentyl, hexyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methoxy, methylenedioxy, ethoxy, propoxy, n-butoxy, hydroxymethyl, hydroxyethyl, methoxymethyl, ethoxymethyl, and trifluoromethoxy;
   R³ is a radical selected from hydrido, methyl, ethyl, isopropyl, *tert*-butyl, isobutyl, pentyl, hexyl, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, and 2-methylphenylthiomethyl;
   R⁴ is a radical selected from hydrido, C₁-C₆ alkyl, and halogen; and
   X' is chloro or bromo.

The compounds obtained by the process according to the present invention are substituted imidazolyl compounds useful in treating inflammation-related disorders.

Compounds obtained by the present invention would be useful for, but not limited to, the treatment of inflammation in a subject, and for treatment of other inflammation-associated disorders, such as, as an analgesic in the treatment of pain and headaches, or as an antipyretic for the treatment of fever. For example, compounds obtained according to the invention would be useful to treat arthritis, including but not limited to rheumatoid arthritis, spondyloarthopathies, gouty arthritis, osteoarthritis, systemic lupus erythematosus and juvenile arthritis. Such compounds of the invention would be useful in the treatment of asthma, bronchitis, menstrual cramps, tendinitis, bursitis, and skin-related conditions such as psoriasis, eczema, burns and dermatitis. Compounds obtained according to the invention also would be useful to treat gastrointestinal conditions such as inflammatory bowel disease, Crohn's disease, gastritis, irritable bowel syndrome and ulcerative colitis, and for the prevention or treatment of cancer, such as colorectal cancer. Compounds obtained by the process of the invention would be useful in treating inflammation in such diseases as vascular diseases, migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury, myocardial ischemia, and the like. The compounds would also be useful in the treatment of ophthalmic diseases such as retinitis, retinopathies, uveitis, conjunctivitis, and of acute injury to the eye tissue. The compounds would also be useful in the treatment of pulmonary inflammation, such as that associated with viral infections and cystic fibrosis. The compounds would also be useful for the treatment of certain central nervous system disorders such as cortical dementias including Alzheimers disease. The compounds obtained according to the invention are useful as anti-inflammatory agents, such as for the treatment of arthritis, with the additional benefit of having significantly less harmful side effects. These compounds would also be useful in the treatment of allergic rhinitis, respiratory distress syndrome, endotoxin shock syndrome, atherosclerosis and central nervous system damage resulting from stroke, ischemia and trauma.

Besides being useful for human treatment, these compounds are also useful for veterinary treatment of mammals, including companion animals and farm animals, such as, but not limited to, horses, dogs, cats, cows, sheep and pigs.
The compounds obtained according to the present invention may also be used in co-therapies, partially or completely, in place of other conventional antiinflammatories, such as together with steroids, NSAIDs, 5-lipoxygenase inhibitors, LTB₄ antagonists and LTA₄ hydrolase inhibitors.

Suitable LTB₄ inhibitors include, among others, ebselen, Bayer Bay-x-1005, Ciba Geigy compound CGS-25019C, Leo Denmark compound ETH-615, Lilly compound LY-293111, Ono compound ONO-4057, Terumo compound TMK-688, Lilly compounds LY-213024, 264086 and 292728, ONO compound ONO-LB457, Searle compound SC-53228, calcitrol, Lilly compounds LY-210073, LY223982, LY233469, and LY255283, ONO compound ONO-LB-448, Searle compounds SC-41930, SC-50605 and SC-51146, and SK&F compound SKF-104493. Preferably, the LTB₄ inhibitors are selected from ebselen, Bayer Bay-x-1005, Ciba Geigy compound CGS-25019C, Leo Denmark compound ETH-615, Lilly compound LY-293111, Ono compound ONO-4057, and Terumo compound TMK-688.

Suitable 5-LO inhibitors include, among others, masoprocol, tenidap, zileuton, pranlukast, tepoxalin, rilopirox, flezelastine hydrochloride, enazadrem phosphate, and bunaprolast.
The present invention preferably includes processes of making compounds which selectively inhibit cyclooxygenase-2 over cyclooxygenase-1. Preferably, the compounds have a cyclooxygenase-2 IC₅₀ equal to or less than about 0.2 µM, and also have a selectivity ratio of cyclooxygenase-2 inhibition over cyclooxygenase-1 inhibition of at least 50, and more preferably of at least 100. Even more preferably, the compounds have a cyclooxygenase-1 IC₅₀ of greater than about 1.0 µM, and more preferably of greater than 10 µM. Such preferred selectivity may indicate an ability to reduce the incidence of common NSAID-induced side effects.

A preferred class of compounds consists of those compounds wherein R⁴ is hydrido.

A family of specific compounds of interest that are obtained according of the present invention consists of compounds and pharmaceutically-acceptable salts thereof as follows:
4-[2-(6-methylpyrindin-2-yl)-4-trifluoromethyl-1H-imidazol-1-yl]benzenesulfonamide;
4-[2-(2-methylthiazol-4-yl)-4-trifluoromethyl-1H-imidazol-1-yl]benzenesulfonamide;
4-[2-(4-methylpyrindin-3-yl)-4-trifluoromethyl-1H-imidazol-1-yl]benzenesulfonamide;
4-[2-(3-methylpyrindin-2-yl)-4-trifluoromethyl-1H-imidazol-1-yl]benzenesulfonamide;
4-[2-(2-thienyl)-4-trifluoromethyl-1H-imidazol-1-yl]benzenesulfonamide;
4-[4-cyano-2-(5-methylpyrindin-3-yl)-1H-imidazol-1-yl]benzenesulfonamide;
4-[2-(2-quinolinyl)-4-trifluoromethyl-1H-imidazol-1-yl]benzenesulfonamide;
4-[2-(3-methoxypyridin-5-yl)-4-(trifluoromethyl)-1H-imidazol-1-yl]benzenesulfonamide;
4-[2-(4-methylpyridin-2-yl)-4-(trifluoromethyl)-1H-imidazol-1-yl]benzenesulfonamide;
4-[2-(5-methylpyridin-3-yl)-4-(trifluoromethyl)-1H-imidazol-1-yl]benzenesulfonamide;
4-[2-(2-methylpyridin-3-yl)-4-(trifluoromethyl)-1H-imidazol-1-yl]benzenesulfonamide;
4-[2-(5-methylpyridin-2-yl)-4-(trifluoromethyl)-1H-imidazol-1-yl]benzenesulfonamide;
4-[2-(4-methylpyridin-2-yl)-4-(trifluoromethyl)-1H-imidazol-1-yl]benzenesulfonamide;
4-[2-(6-methoxypyridin-2-yl)-4-(trifluoromethyl)-1H-imidazol-1-yl]benzenesulfonamide;
4-[2-(5-methoxypyridin-2-yl)-4-(trifluoromethyl)-1H-imidazol-1-yl]benzenesulfonamide;
4-[2-(4-methoxypyridin-2-yl)-4-(trifluoromethyl)-1H-imidazol-1-yl]benzenesulfonamide;
4-[2-(5-methoxypyridin-3-yl)-4-(trifluoromethyl)-1H-imidazol-1-yl]benzenesulfonamide;
4-[4-methyl-2-(3-pyridinyl)-1H-imidazol-1-yl]benzenesulfonamide;
4-[2-(pyridin-3-yl)-4-(trifluoromethyl)-1H-imidazol-1-yl]benzenesulfonamide;
4-[2-[6-(methylthio)pyridin-3-yl]-4-trifluoromethyl)-1H-imidazol-1-yl]benzenesulfonamide;
4-[4-(difluoromethyl)-2-(3-pyridinyl)-1H-imidazol-1-yl]benzenesulfonamide;
4-[2-(6-methylpyridin-3-yl)-4-(trifluoromethyl)-1H-imidazol-1-yl]benzenesulfonamide; and
pharmaceutically acceptable salts thereof.

A family of specific compounds of particular interest that are obtained according to the present invention consists of compounds and pharmaceutically-acceptable salts thereof as follows:
4-[2-(pyrindin-3-yl)-4-[[(methylphenyl)thio]methyl]-1H-imidazol-1-yl]benzenesulfonamide;
4-[2-(6-methylpyrindin-2-yl)-4-trifluoromethyl-1H-imidazol-1-yl]benzenesulfonamide;
4-[2-(4-methylpyrindin-3-yl)-4-trifluoromethyl-1H-imidazol-1-yl]benzenesulfonamide;
4-[2-(3-methylpyrindin-2-yl)-4-trifluoromethyl-1H-imidazol-1-yl]benzenesulfonamide;
4-[2-(2-thienyl)-4-trifluoromethyl-1H-imidazol-1-yl]benzenesulfonamide;
4-[4-difluoromethyl-2-(pyrindin-3-yl)-1H-imidazol-1-yl]benzenesulfonamide;
4-[4-cyano-2-(pyrindin-3-yl)-1H-imidazol-1-yl]benzenesulfonamide;
4-[4-cyano-2-(5-methylpyrindin-3-yl)-1H-imidazol-1-yl]benzenesulfonamide;
4-[2-(2-quinolinyl)-4-trifluoromethyl-1H-imidazol-1-yl]benzenesulfonamide;
4-[2-(1-methyl-1H-pyrazol-4-yl)-4-trifluoromethyl-1H-imidazol-1-yl]benzenesulfonamide;
4-[2-(1-methyl-1H-imidazol-4-yl)-4-trifluoromethyl-1H-imidazol-1-yl]benzenesulfonamide;
4-[2-(1-methyl-1H-imidazol-5-y1)-4-trifluoromethyl-1H-imidazol-1-yl]benzenesulfonamide;
4-[2-(1-methyl-1H-imidazol-2-yl)-4-trifluoromethyl-1H-imidazol-1-yl]benzenesulfonamide;
4-[2-(4-methylthiazol-2-yl)-4-trifluoromethyl-1H-imidazol-1-yl]benzenesulfonamide;
4-[2-(2-methylthiazol-5-yl)-4-trifluoromethyl-1H-imidazol-1-yl]benzenesulfonamide;
4-[2-(S-methylisoxazol-3-yl)-4-trifluoromethyl-1H-imidazol-1-yl]benzenesulfonamide;
4-[2-(5-pyrimidinyl)-4-trifluoromethyl-1H-imidazol-1-yl]benzenesulfonamide;
4-[2-(pyrazin-2-yl)-4-(trifluoromethyl)-1H-imidazol-1-yl]benzenesulfonamide;
4-[2-(quinol-3-yl)-4-(trifluoromethyl)-1H-imidazol-1-yl]benzenesulfonamide; and
pharmaceutically acceptable salts thereof.

Compounds obtained according to the present invention, especially where R² is pyridyl, may form N-oxides, which may be active forms or prodrugs which would be converted to compounds obtained according to the present invention *in vivo.*

Compounds obtained according to the present invention would also be capable of inhibiting cytokines, such as TNF, IL-1, IL-6, and IL-8. As such, the compounds can be used in the manufacture of a medicament or in a method for the treatment for the prophylactic or therapeutic treatment of diseases mediated by cytokines, such as TNF, IL-1, IL-6, and IL-8.

The term "hydrido" denotes a single hydrogen atom (H). This hydrido radical may be attached, for example, to an oxygen atom to form a hydroxyl radical, or two hydrido radicals may be attached to a carbon atom to form a methylene (-CH₂-) radical. Where used, either alone or within other terms such as "haloalkyl", "alkylsulfonyl", "alkoxyalkyl" and "hydroxyalkyl", the term "alkyl" embraces linear or branched radicals having one to about twenty carbon atoms or, preferably, one to about twelve carbon atoms. More preferred alkyl radicals are "lower alkyl" radicals having one to about ten carbon atoms. Most preferred are lower alkyl radicals having one to about six carbon atoms. Examples of such radicals include methyl, ethyl, n-propyl, isopropyl, n-butyl, isobutyl, sec-butyl, *tert*-butyl, pentyl, isoamyl, hexyl and the like. The term "halo" means halogens such as fluorine, chlorine, bromine or iodine. The term "haloalkyl" embraces radicals wherein any one or more of the alkyl carbon atoms is substituted with halo as defined above. Specifically embraced are monohaloalkyl, dihaloalkyl and polyhaloalkyl radicals. A monohaloalkyl radical, for one example, may have either an iodo, bromo, chloro or fluoro atom within the radical. Dihalo and polyhaloalkyl radicals may have two or more of the same halo atoms or a combination of different halo radicals. "Lower haloalkyl" embraces radicals having one to six carbon atoms. Examples of haloalkyl radicals include fluoromethyl, difluoromethyl, trifluoromethyl, chloromethyl, dichloromethyl, trichloromethyl, trichloromethyl, pentafluoroethyl, heptafluoropropyl, difluorochloromethyl, dichlorofluoromethyl, difluoroethyl, difluoropropyl, dichloroethyl and dichloropropyl. The term "hydroxyalkyl" embraces linear or branched alkyl radicals having one to about ten carbon atoms any one of which may be substituted with one or more hydroxyl radicals. More preferred hydroxyalkyl radicals are "lower hydroxyalkyl" radicals having one to six carbon atoms and one or more hydroxyl radicals. Examples of such radicals include hydroxymethyl, hydroxyethyl, hydroxypropyl, hydroxybutyl and hydroxyhexyl. The terms "alkoxy" and "alkoxyalkyl" embrace linear or branched oxy-containing radicals each having alkyl portions of one to about ten carbon atoms. More preferred alkoxy radicals are "lower alkoxy" radicals having one to six carbon atoms. Examples of such radicals include methoxy, ethoxy, propoxy, butoxy and *tert*-butoxy. The term "alkoxyalkyl" embraces alkyl radicals having one or more alkoxy radicals attached to the alkyl radical, that is, to form monoalkoxyalkyl and dialkoxyalkyl radicals. More preferred alkoxyalkyl radicals are "lower alkoxyalkyl" radicals having one to six carbon atoms and one or two alkoxy radicals. Examples of such radicals include methoxymethyl, methoxyethyl, ethoxyethyl, methoxybutyl and methoxypropyl. The "alkoxy" or "alkoxyalkyl" radicals may be further substituted with one or more halo atoms, such as fluoro, chloro or bromo, to provide "haloalkoxy" or "haloalkoxyalkyl" radicals. More preferred haloalkoxy radicals are "lower haloalkoxy" radicals having one to six carbon atoms and one or more halo radicals. Examples of such radicals include fluoromethoxy, chloromethoxy, trifluoromethoxy, trifluoroethoxy, fluoroethoxy and fluoropropoxy. The term "cyanoalkyl" embraces radicals having a cyano or nitrile (-CN) radical attached to an alkyl radical as described above. More preferred cyanoalkyl radicals are "lower cyanoalkyl" radicals having one to six carbon atoms. Examples of such lower cyanoalkyl radicals include cyanomethyl, cyanopropyl, cyanoethyl and cyanobutyl. The term "cycloalkyl" embraces saturated carbocyclic radicals having three to twelve carbon atoms. More preferred cycloalkyl radicals are "lower cycloalkyl" radicals having three to about eight carbon atoms. Examples of such radicals include cyclopropyl, cyclobutyl, cyclopentyl and cyclohexyl. The term "cycloalkenyl" embraces unsaturated cyclic radicals having three to ten carbon atoms. More preferred cycloalkenyl radicals are "lower cycloalkenyl" radicals having about five to about eight carbon atoms. Examples of such radicals include cyclobutenyl, cyclopentenyl, cyclohexenyl and cycloheptenyl. The term "aryl", alone or in combination, means a carbocyclic aromatic system containing one, two or three rings wherein such rings may be attached together in a pendent manner or may be fused. The term "aryl" embraces aromatic radicals such as phenyl, naphthyl, tetrahydronaphthyl, indane and biphenyl. Such aryl radicals may be substituted at a substitutable position with one or more substituents selected from halo, alkylthio, alkylsulfinyl, alkyl, cyano, haloalkyl, hydroxyl, alkoxy, hydroxyalkyl and haloalkoxy. The terms "heterocyclic" and "heterocyclo" embraces saturated, partially saturated and unsaturated heteroatom-containing ring-shaped radicals, where the heteroatoms may be selected from nitrogen, sulfur and oxygen. Examples of saturated heterocyclo radicals include saturated 3 to 6-membered heteromonocylic group containing 1 to 4 nitrogen atoms [e.g. pyrrolidinyl, imidazolidinyl, piperidino, piperazinyl, etc.]; saturated 3 to 6-membered heteromonocyclic group containing 1 to 2 oxygen atoms and 1 to 3 nitrogen atoms [e.g. morpholinyl, etc.]; and saturated 3 to 6-membered heteromonocyclic group containing 1 to 2 sulfur atoms and 1 to 3 nitrogen atoms [e.g., thiazolidinyl, etc.]. Examples of partially saturated heterocyclic radicals include dihydrothiophene, dihydropyran, dihydrofuran and dihydrothiazole. The term "heteroaryl" embraces unsaturated heterocyclic radicals. Examples of "heteroaryl" radicals include unsaturated 3 to 6 membered heteromonocyclic group containing 1 to 4 nitrogen atoms, for example, pyrrolyl, pyrrolinyl, imidazolyl, pyrazolyl, 2-pyridyl, 3-pyridyl, 4-pyridyl, pyrimidyl, pyrazinyl, pyridazinyl, triazolyl [e.g., 4H-1,2,4-triazolyl, 1H-1,2,3-triazolyl, 2H-1,2,3-triazolyl, etc.] tetrazolyl [e.g. 1H-tetrazolyl, 2H-tetrazolyl, etc.], etc.; unsaturated condensed heterocyclic group containing 1 to 5 nitrogen atoms, for example, indolyl, isoindolyl, indolizinyl, benzimidazolyl, quinolyl (quinolinyl), isoquinolyl (isoquinolinyl), indazolyl, benzotriazolyl, tetrazolopyridazinyl [e.g., tetrazolo [1,5-b]pyridazinyl, etc.], etc.; unsaturated 3 to 6-membered heteromonocyclic group containing an oxygen atom, for example, pyranyl, 2-furyl, 3-furyl, etc.; unsaturated 3 to 6-membered heteromonocyclic group containing a sulfur atom, for example, 2-thienyl, 3-thienyl, etc.; unsaturated 3- to 6-membered heteromonocyclic group containing 1 to 2 oxygen atoms and 1 to 3 nitrogen atoms, for example, oxazolyl, isoxazolyl, oxadiazolyl [e.g., 1,2,4-oxadiazolyl, 1,3,4-oxadiazolyl, 1,2,5-oxadiazolyl, etc.] etc.; unsaturated condensed heterocyclic group containing 1 to 2 oxygen atoms and 1 to 3 nitrogen atoms [e.g. benzoxazolyl, benzoxadiazolyl, etc.]; unsaturated 3 to 6-membered heteromonocyclic group containing 1 to 2 sulfur atoms and 1 to 3 nitrogen atoms, for example, thiazolyl, thiadiazolyl [e.g., 1,2,4- thiadiazolyl, 1,3,4-thiadiazolyl, 1,2,5-thiadiazolyl, etc.] etc.; unsaturated condensed heterocyclic group containing 1 to 2 sulfur atoms and 1 to 3 nitrogen atoms [e.g., benzothiazolyl, benzothiadiazolyl, etc.] and the like. The term also embraces radicals where heterocyclic radicals are fused with aryl radicals. Examples of such fused bicyclic radicals include benzofuran, benzothiophene, and the like. Said heterocyclo may be substituted at a substitutable position with one or more substituents selected from halo, alkylthio, alkylsulfinyl, alkyl, cyano, haloalkyl, hydroxyl, alkoxy, hydroxyalkyl and haloalkoxy. More preferred heteroaryl radicals include five to six membered heteroaryl radicals. The term "heterocycloalkyl" embraces heterocyclic-substituted alkyl radicals. More preferred heterocycloalkyl radicals are "lower heterocycloalkyl" radicals having one to six carbon atoms and a heterocyclic radical. Examples include such radicals as pyrrolidinylmethyl. The term "heteroarylalkyl" embraces heteroaryl-substituted alkyl radicals. More preferred heteroarylalkyl radicals are "lower heteroarylalkyl" radicals having one to six carbon atoms and a heteroaryl radical. Examples include such heteroarylalkyl radicals such as pyridylmethyl and thienylmethyl. The term "alkylthio" embraces radicals containing a linear or branched alkyl radical, of one to about ten carbon atoms attached to a divalent sulfur atom. More preferred alkylthio radicals are "lower alkylthio" radicals having alkyl radicals of one to six carbon atoms. Examples of such lower alkylthio radicals are methylthio, ethylthio, propylthio, butylthio and hexylthio. The term "alkylthioalkyl" embraces alkylthio radicals attached to an alkyl radical. More preferred alkylthioalkyl radicals are "lower alkylthioalkyl" radicals having alkyl radicals of one to six carbon atoms and an alkylthio radical as described above. Examples of such radicals include methylthiomethyl. The term "arylthio" embraces radicals containing an aryl radical, attached to a divalent sulfur atom, such as a phenylthio radical. The term "arylthioalkyl" embraces arylthio radicals attached to an alkyl radical. More preferred arylthioalkyl radicals are "lower arylthioalkyl" radicals having alkyl radicals of one to six carbon atoms and an arylthio radical as described above. Examples of such radicals include phenylthiomethyl, where the phenyl radical may be substituted as described above. The term "alkylsulfinyl" embraces radicals containing a linear or branched alkyl radical, of one to ten carbon atoms, attached to a divalent -S(=O)- radical. More preferred alkylsulfinyl radicals are "lower alkylsulfinyl" radicals having one to six carbon atoms. Examples of such lower alkylsulfinyl radicals include methylsulfinyl, ethylsulfinyl, butylsulfinyl and hexylsulfinyl. The term "sulfonyl", whether used alone or linked to other terms such as alkylsulfonyl, denotes respectively divalent radicals -SO₂-. "Alkylsulfonyl" embraces alkyl radicals attached to a sulfonyl radical, where alkyl is defined as above. More preferred alkylsulfonyl radicals are "lower alkylsulfonyl" radicals having one to six carbon atoms. Examples of such lower alkylsulfonyl radicals include methylsulfonyl, ethylsulfonyl and propylsulfonyl. The "alkylsulfonyl" radicals may be further substituted with one or more halo atoms, such as fluoro, chloro or bromo, to provide "haloalkylsulfonyl" radicals. More preferred haloalkylsulfonyl radicals are "lower haloalkylsulfonyl" radicals having one or more halo atoms attached to lower alkylsulfonyl radicals as described above. Examples of such lower haloalkylsulfonyl radicals include fluoromethylsulfonyl, trifluoromethylsulfonyl and chloromethylsulfonyl. The term "arylsulfonyl" embraces aryl radicals as defined above, attached to a sulfonyl radical. Examples of such radicals include phenylsulfonyl. The terms "sulfamyl", "aminosulfonyl" and "sulfonamidyl" denotes NH₂O₂S-. The term "acyl" denotes a radical provided by the residue after removal of hydroxyl from an organic acid. Examples of such acyl radicals include formyl, alkanoyl and aroyl radicals. The alkanoyl radicals may be substituted or unsubstituted, such as formyl, acetyl, propanoyl, butanoyl, isobutanoyl, valeryl, isovaleryl, pivaloyl, hexanoyl or the like. The terms "carboxy" or "carboxyl", whether used alone or with other terms, such as "carboxyalkyl", denotes -CO₂H. The term "carbonyl", whether used alone or with other terms, such as "alkoxycarbonyl", denotes -(C=O)-. The term "alkoxycarbonyl" means a radical containing an alkoxy radical, as defined above, attached via an oxygen atom to a carbonyl radical. Preferably, "lower alkoxycarbonyl" embraces alkoxy radicals having one to six carbon atoms. Examples of such "lower alkoxycarbonyl" ester radicals include substituted or unsubstituted methoxycarbonyl, ethoxycarbonyl, propoxycarbonyl, butoxycarbonyl and hexyloxycarbonyl. The term "aralkyl" embraces aryl-substituted alkyl radicals. Preferable aralkyl radicals are "lower aralkyl" radicals having aryl radicals attached to alkyl radicals having one to six carbon atoms. Examples of such phenylalkyl radicals include benzyl, diphenylmethyl, triphenylmethyl, phenylethyl and diphenylethyl. The aryl in said aralkyl radicals may be substituted at a substitutable position with one or more substituents selected from halo, alkylthio, alkylsulfinyl, alkyl, cyano, haloalkyl, hydroxyl, alkoxy, hydroxyalkyl and haloalkoxy. The terms benzyl and phenylmethyl are interchangeable. The terms "alkylcarbonyl", "arylcarbonyl" and "aralkylcarbonyl" include radicals having alkyl, aryl and aralkyl radicals, respectively, as defined above, attached to a carbonyl radical. More preferred alkylcarbonyl radicals are "lower alkylcarbonyl" radicals having one to six carbon atoms. Examples of such radicals include methylcarbonyl and ethylcarbonyl. More preferred aralkylcarbonyl radicals are "lower aralkylcarbonyl" radicals having aryl radicals attached to alkyl radicals having one to six carbon atoms. Examples of such aralkylcarbonyl radicals include benzylcarbonyl. An example of an arylcarbonyl radical is phenylcarbonyl. The term "alkoxycarbonylalkyl" embraces radicals having "alkoxycarbonyl", as defined above attached to an alkyl radical. More preferred alkoxycarbonylalkyl radicals are "lower alkoxycarbonylalkyl" having lower alkoxycarbonyl radicals as defined above attached to one to six carbon atoms. Examples of such lower alkoxycarbonylalkyl radicals include methoxycarbonylmethyl. The term "haloalkylcarbonyl" embraces radicals having a haloalkyl radical as described above attached to a carbonyl radical. More preferred radicals are "lower haloalkylcarbonyl" radicals where lower haloalkyl radicals, as described above are attached to a carbonyl radical. The term "carboxyalkyl" embraces radicals having a carboxy radical as defined above, attached to an alkyl radical. More preferred carboxyalkyl radicals are "lower carboxyalkyl" radicals having one or more carboxy radicals attached to an alkyl radical having one to six carbon atoms. The term "heteroaralkyl" embraces heteroaryl-substituted alkyl radicals. More preferred heteroaralkyl radicals are "lower heteroaralkyl" radicals having five to six membered heteroaryl radicals attached to one to six carbon atoms. Examples of such radicals include pyridylmethyl, quinolylmethyl, thienylmethyl, furylethyl and quinolylethyl. The heteroaryl in said heteroaralkyl may be additionally substituted with halo, alkyl, alkoxy, halkoalkyl and haloalkoxy. The term "aryloxy" embraces aryl radicals, as defined above, attached to an oxygen atom. Examples of such radicals include phenoxy. The term "heteroaryloxy" embraces heteroaryl radicals as defined above attached to an oxygen radical. More preferred heteroaryloxy radicals are "lower heteroaryloxy" radicals having five to six membered heteroaryl radicals. The term "aralkoxy" embraces oxy-containing aralkyl radicals attached through the oxygen atom to other radicals. The term "aralkoxyalkyl" embraces alkyl radicals having one or more aralkoxy radicals attached to the alkyl radical, that is, to form monoaralkyloxyalkyl and diaralkyloxyalkyl radicals. The "aralkoxy" or "aralkoxyalkyl" radicals may be further substituted on the aryl ring portion of the radical. More preferred aralkoxyalkyl radicals are "lower aralkoxyalkyl" having an alkoxy attached to one to six carbon atoms. Examples of lower aralkoxyalkyl radicals include benzyloxymethyl. The term "cycloalkylthio" embraces radicals containing a cycloalkyl radical, of three to about ten carbon atoms attached to a divalent sulfur atom. More preferred cycloalkylthio radicals are "lower cycloalkylthio" radicals having cycloalkyl radicals of four to six carbon atoms. Examples of such lower cycloalkylthio radicals are cyclobutylthio, cyclopentylthio and cyclohexylthio. The term "cycloalkylthioalkyl" embraces radicals containing a cycloalkylthio radical, as described above, attached to an alkyl radical. More preferred cycloalkylthioalkyl radicals are "lower cycloalkylthioalkyl" radicals having cycloalkyl radicals of four to six carbon atoms and alkyl radicals of one to six carbons. The term "cycloalkylsulfonyl" embraces radicals containing a cycloalkyl radical, of three to about ten carbon atoms attached to a divalent sulfonyl radical. More preferred cycloalkylsulfonyl radicals are "lower cycloalkylsulfonyl" radicals having cycloalkyl radicals of four to six carbon atoms. Examples of such lower cycloalkylsulfonyl radicals are cyclobutylsulfonyl, cyclopentylsulfonyl and cyclohexylsulfonyl. The term "cycloalkylsulfonylalkyl" embraces radicals containing a cycloalkylsulfonyl radical, as described above, attached to an alkyl radical. More preferred cycloalkylsulfonylalkyl radicals are "lower cycloalkylsulfonylalkyl" radicals having cycloalkyl radicals of four to six carbon atoms and alkyl radicals of one to six carbons. The term "cycloalkyloxy" embraces radicals containing a cycloalkyl radical, of three to about ten carbon atoms attached to a divalent oxygen atom. More preferred cycloalkyloxy radicals are "lower cycloalkyloxy" radicals having cycloalkyl radicals of four to six carbon atoms. Examples of such lower cycloalkyloxy radicals are cyclobutyloxy, cyclopentyloxy and cyclohexyloxy. The term "cycloalkyloxyalkyl" embraces radicals containing a cycloalkyloxy radical, as described above, attached to an alkyl radical. More preferred cycloalkyloxyalkyl radicals are "lower cycloalkyloxyalkyl" radicals having cycloalkyl radicals of four to six carbon atoms and alkyl radicals of one to six carbons. The term "heteroarylthio" embraces radicals having heteroaryl radicals attached to a sulfur radical. More preferred heteroarylthio radicals are "lower heteroarylthio" radicals having five to six membered heteroaryl radicals. Examples of such radicals include 2-furylthio, 2-thienylthio, 3-thienylthio, 4-pyridylthio and 3-pyridylthio. The term "heteroarylalkylthio" denotes radicals having an heteroaryl radical attached to an alkylthio radical. More preferred heteroarylalkylthio radicals are "lower heteroarylalkylthio" radicals having heteroaryl radicals attached to lower alkylthio radicals as described above. Examples of such radicals include furylmethylthio and quinolylmethylthio. The term "heteroarylalkylthioalkyl" denotes radicals having an heteroaryl radical attached to an alkylthio radical further attached through the sulfur atom to an alkyl radical. More preferred heteroarylalkylthioalkyl are "lower heteroarylalkylthioalkyl" radicals having lower heteroarylalkyl radicals as described above. Examples of such radicals include furylmethylthiomethyl and quinolylmethylthioethyl. The term "heteroarylthioalkyl" denotes radicals having an heteroaryl radical attached to a sulfur atom further attached through the sulfur atom to an alkyl radical. More prefered heteroarylthioalkyl radicals are "lower heteroarylthioalkyl" having lower heteroarylthio radicals as described above. Examples of such radicals include thienylthiomethyl and pyridylthiohexyl. The term "aralkylthio" embraces radicals having aralkyl radicals attached to a bridging sulfur atom. More preferred aralkylthio radicals are "lower aralkylthio" radicals having the aryl radicals attached to one to six carbon atoms. Examples of such radicals include benzylthio and phenylethylthio. The term "aralkylthioalkyl" embraces radicals having aralkyl radicals attached to alkyl radicals through a bridging sulfur atom. More preferred aralkylthioalkyl radicals are "lower aralkylthioalkyl" radicals having the aralkylthio radicals attached to one to six carbon atoms. Examples of such radicals include benzylthiomethyl and phenylethylthiomethyl. The term "heteroaryloxyalkyl" denotes radicals having an heteroaryl radical attached to an oxygen atom further attached through the oxygen atom to an alkyl radical. More preferred heteroaryloxyalkyl radicals are "lower heteroaryloxyalkyl" radicals having five to six membered heteroaryl radicals. Examples of such radicals include furyloxyethyl, pyridyloxymethyl and thienyloxyhexyl. The term "aminoalkyl" embraces alkyl radicals substituted with amino radicals. More preferred aminoalkyl radicals are "lower aminoalkyl" having one to six carbon atoms. Examples include aminomethyl, aminoethyl and aminobutyl. The term "alkylaminoalkyl" embraces aminoalkyl radicals having the nitrogen atom substituted with at least one alkyl radical. More preferred alkylaminoalkyl radicals are "lower alkylaminoalkyl" having one to six carbon atoms attached to a lower aminoalkyl radical as described above. The term "alkylamino" denotes amino groups which have been substituted with one or two alkyl radicals. More preferred alkylamino radicals are "lower alkylamino" radicals having one or two alkyl radicals of one to six carbon atoms, attached to a nitrogen atom. Suitable "alkylamino" may be mono or dialkylamino such as N-methylamino, N-ethylamino, N,N-dimethylamino, N,N-diethylamino and the like. TThe term "aminocarbonyl" denotes an amide group of the formula -C(=O)NH₂. he term "alkylaminocarbonyl" embraces alkylamino radicals, as described above, to a carbonyl radical. More preferred alkylaminocarbonyl radicals are "lower alkylaminocarbonyl" having lower alkylamino radicals, as described above, attached to a carbonyl radical. Examples of such radicals include N-methylaminocarbonyl and N,N-dimethylaminocarbonyl. The term "arylamino" denotes amino groups which have been substituted with one or two aryl radicals, such as N-phenylamino. The "arylamino" radicals may be further substituted on the aryl ring portion of the radical. The terms "N-arylaminoalkyl" and "N-aryl-N-alkylaminoalkyl" denote amino groups which have been substituted with one aryl radical or one aryl and one alkyl radical, respectively, and having the amino group attached to an alkyl radical. More preferred arylaminoalkyl radicals are "lower arylaminoalkyl" having the arylamino radical attached to one to six carbon atoms. Examples of such radicals include N-phenylaminomethyl and N-phenyl-N-methylaminomethyl. The term "alkylaminocarbonylalkyl" denotes an alkylaminocarbonyl group which is attached to an alkyl radical. More preferred are "lower alkylaminocarbonylalkyl" having lower alkylaminocarbonyl radicals as described above attached to one to six carbon atoms. The term "aryloxyalkyl" embraces alkyl radicals having one or more aryloxy radicals, aryl radicals attached to a divalent oxygen atom, attached to the alkyl radical, that is, to form monoaryloxyalkyl and diaryloxyalkyl radicals. The more preferred aryloxyalkyl radicals are "lower aryloxyalkyl" radicals having aryloxy radicals attached to one to six carbon atoms. Examples include phenoxymethyl. The term "heteroarylalkoxy" embraces radicals having one or more heteroaryl radicals attached to an alkoxy radical. More preferred heteroarylalkoxy radicals are "lower heteroarylalkoxy" radicals having five to six membered heteroaryl radicals. Examples of such radicals include 2-thienylmethoxy, 3-thienylmethoxy, 2-furylmethoxy, 3-furylmethoxy and 2-pyridylmethoxy, 3-pyridylmethoxy, 4-pyridylmethoxy. The term "heteroarylalkoxyalkyl" embraces alkyl radicals having one or more heteroaryl radicals attached to an alkoxy radical, further attached to the alkyl radical. More preferred heteroarylalkoxyalkyl radicals are "lower heteroarylalkoxyalkyl radicals having five to six membered heteroaryl radicals. Examples of such radicals include 2-thienylmethoxymethyl. The term "azidoalkyl" denotes alkyl radicals substituted with azido groups (-N₃). More preferred azidoalkyl radicals are "lower azidoalkyl" having one to six carbon atoms. Examples include azidomethyl, azidoethyl and aminopropyl. Also included in the family of compounds obtained according to I the invention are the stereoisomers thereof. Compounds obtained according to the present invention can possess one or more asymmetric carbon atoms and are thus capable of existing in the form of optical isomers as well as in the form of racemic or nonracemic mixtures thereof. Accordingly, some of the compounds obtained according to this invention may be present in racemic mixtures which are also included in this invention. The optical isomers can be obtained by resolution of the racemic mixtures according to conventional processes, for example by formation of diastereoisomeric salts by treatment with an optically active acid or base. Examples of appropriate acids are tartaric, diacetyltartaric, dibenzoyltartaric, ditoluoyltartaric and camphorsulfonic acid and then separation of the mixture of diastereoisomers by crystallization followed by liberation of the optically active bases from these salts. A different process for separation of optical isomers involves the use of a chiral chromatography column optimally chosen to maximize the separation of the enantiomers. Still another available method involves synthesis of covalent diastereoisomeric molecules by reacting an amine functionality of precursors to compounds obtained according to the present invention with an optically pure acid in an activated form or an optically pure isocyanate. Alternatively, diastereomeric derivatives can be prepared by reacting a carboxyl functionality of precursors to compounds of Formula I with an optically pure amine base. The synthesized diastereoisomers can be separated by conventional means such as chromatography, distillation, crystallization or sublimation, and then hydrolyzed to deliver the enantiomerically pure compound. The optically active compounds obtained according to the present invention can likewise be obtained by utilizing optically active starting materials. These isomers may be in the form of a free acid, a free base, an ester or a salt. Also included in the family of compounds obtained according to the present invention are the pharmaceutically-acceptabie salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds obtained according to the present invention may be prepared from an inorganic acid or from an organic acid. Examples of such inorganic acids are hydrochloric, hydrobromic, hydroiodic, nitric, carbonic, sulphuric and phosphoric acid. Appropriate organic acids may be selected from aliphatic, cycloaliphatic, aromatic, araliphatic, heterocyclic, carboxylic and sulfonic classes of organic acids, example of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, salicylic, *p*-hydroxybenzoic, phenylacetic, mandelic, embonic (pamoic), methanesulfonic, ethylsulfonic, benzenesulfonic, pantothenic, toluenesulfonic, 2-hydroxyethanesulfonic, sulfanilic, stearic, cyclohexylaminosulfonic, algenic, β-hydroxybutyric, salicylic, galactaric and galacturonic acid. Suitable pharmaceutically-acceptable base addition salts of compounds obtained according to the present invention include metallic salts made from aluminum, calcium, lithium, magnesium, potassium, sodium and zinc or organic salts made from N,N'-dibenzylethylenediamine, choline, chloroprocaine, diethanolamine, ethylenediamine, meglumine (N-methylglucamine) and procaine. All of these salts may be prepared by conventional means from the corresponding compound of Formula I by reacting, for example, the appropriate acid or base with the compound obtained according to the present invention.

Racemic alcohol containing compounds may be resolved to their single enantiomers by the following procedure. Treatment of the racemic alcohols with an acetylating agent, such.as vinyl acetate or isopropenyl acetate, in the presence of an appropriate enzyme results in the selective acetylation of one of the constituent enantiomeric alcohols, leading to a crude product consisting of essentially enantiomerically pure alcohol. Appropriate enzymes include, but are not limited to, lipases (such as AMANO Lipase PS30), cholinesterases and proteases. The reaction may be monitored to complete acetylation of one of the enantiomers using HPLC. The enantiomerically pure alcohol may be separated from enantiomerically pure acetate by column chromatography. Saponification of the acetate using aqueous base provides the other enantiomerically pure alcohol.

Alternatively, alcohols can be resolved via procedures outlined in E. Eliel and S. Wilen, *Stereochemistry of Organic compounds,* 337-340 (1994).

### GENERAL SYNTHETIC PROCEDURES

In the following, the methods according to the present invention are illustrated with reference to Scheme I, wherein the R¹-R⁴ substituents are as defined above, except where further noted.

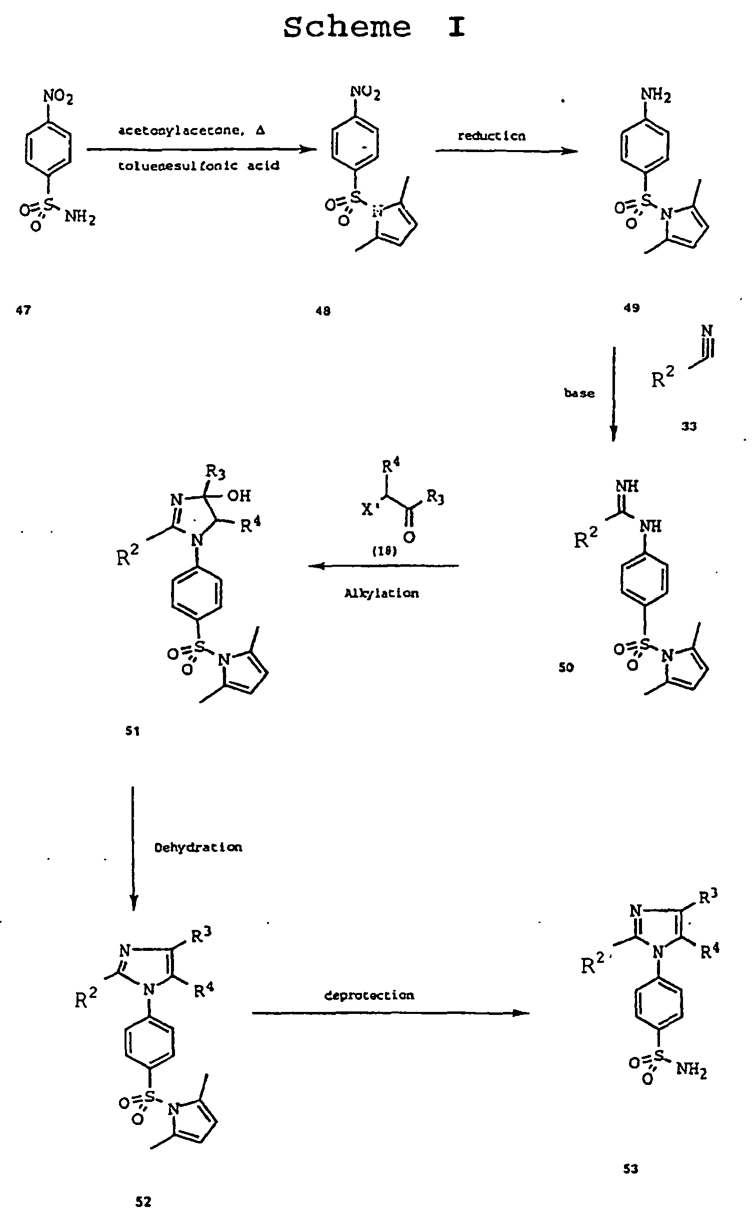

Synthetic Scheme I describes the method of forming 1-aryl-2-pyridyl-imidazoles 53 from 4-nitrobenzenesulfonamide 47. Protection of 4-nitrobenzenesulfonamide 47, such as by reaction with acetonylacetone with p-toluenesulfonic acid as catalyst in a solvent such as toluene, forms the protected pyrrolylsulfonyl 48. A preferred protecting agent is 2,5-lower alkyl pyrrole, and more preferred is 2,5-dimethyl pyrrole. Reduction of the nitro compound 48, such as by Raney Nickel-catalyzed hydrogenation, yields the protected benzenamine 49. Amidine 50 is synthesized by reaction of benzenamine 49 first with a suitable base, and then with nitrile 33. Examples of suitable bases include sodium bis(trimethylsilyl)amide, sodium hydride, sodium methoxide, n-butyllithium and lithium diisopropylamide. This reaction may be run in solvents such as dimethyl sulfoxide, tetrahydrofuran, dimethoxyethane, methanol, or the like. The reaction of amidine 50 with a 2-halo-ketone derivative 18 (X' = Br or Cl) in the presence of bases such as sodium bicarbonate, potassium carbonate, sodium carbonate, potassium bicarbonate or N,N'-diisopropylethylamine gives the hydroxyimidazole 51. Some of the suitable solvents for this reaction are isopropanol, acetone and dimethylformamide. The reaction may be carried out at 20 to 90°C. The intermediate 51 is dehydrated in the presence of an acid catalyst such as 4-toluenesulfonic acid to give the protected 1-(4-sulfonyl)aryl-imidazoles 52. Suitable solvents for this dehydration step are e.g., toluene, xylene and benzene. Acid deprotection of 52 such as with aqueous trifluoroacetic acid (TFA) at reflux temperature produces the sulfonamides 53.

The following related applications are currently pending: International Application PCT/US95/09506, Patent Application Serial No. 08/464,154, and Patent Application Serial No. 08/282,395.

The following example contains detailed descriptions of the methods according to the present invention. These detailed descriptions fall within the scope, and serve to exemplify, the above described General Synthetic Procedures which form part of the invention. These detailed descriptions are presented for illustrative purposes only and are not intended as a restriction on the scope of the invention. All parts are by weight and temperatures are in Degrees centigrade unless otherwise indicated. All compounds showed NMR spectra consistent with their assigned structures. In some cases, the assigned structures were confirmed by nuclear Overhauser effect (NOE) experiments.

Alternative processes for the synthesis of heterocyclo-substituted imidazoles which are suitable for the treatment of inflammation are disclosed in the international patent application WO-A 96/03388.

Furthermore, general methods for the synthesis of imidazole compounds are disclosed in Houben-Weyl, "Methoden der Organischen Chemie" Vol. E8C, "Hetarene III, Teil 3", Georg Thieme Verlag, Stuttgart, 1994, pages 26-30 (using α-hydroxy or α-haloketones and amidines). Additional procedures for the synthesis of the amidines employed in the process according to the present invention are disclosed in J.-A. Gautier, et al., Bulletin de la Societe Chimique de France, No. 1, 1970, pages 200-207. The amidines are obtained from nitriles and aromatic amines in a liquid ammonia solvent.

The following imidazole derivatives in Table I may be obtained according to the procedures of the present invention.

The sulfonamide derivatives were synthesized from the corresponding sulfones using experimental procedure given for Examples 26-27, and 45 of EP 0 880 504 B1 and from protected nitrobenzenes as in Example 1 below.

**Table I: Characterization of Compounds**

| | | | | | | | | | | | |
|---|---|---|---|---|---|---|---|---|---|---|---|
| 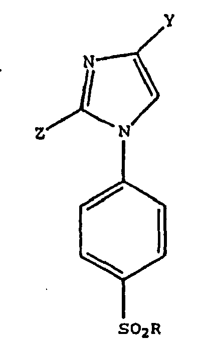 | | | | | | | | | | | |

| R | Y | Z | mp DSC (°C) | Elemental Analysis | | | | | | | |
|---|---|---|---|---|---|---|---|---|---|---|---|
| | | | | Calc'd | | | | Found | | | |
| | | | | C | H | N | S | C | H | N | S |
| NH₂ | CF₃ | 3-methoxy-5-pyridyl | 262-264 | 48.24 | 3.29 | 14.06 | 8.05 | 48.49 | 3.34 | 13.55 | 8.01 |
| | | | | | | | | | | | |
| NH₂ | H | 5-methyl-2-pyridyl | 216 | 50.26 | 3.43 | 14.65 | | 50.58 | 3.49 | 14.50 | |
| NH₂ | CF₃ | 6-methyl-2-pyridyl | 260 | 50.26 | 3.43 | 14.65 | | 50.33 | 3.60 | 14.39 | |

**Table IV: Characterization of Compounds**

| R | Y | Z | mp DSC (°C) | Elemental Analysis | | | | | | | |
|---|---|---|---|---|---|---|---|---|---|---|---|
| | | | | Calc'd | | | | Found | | | |
| | | | | C | H | N | S | C | H | N | S |
| NH₂ | CF₃ | 2-methyl-4-thiazolyl | 250.8 | 43.30 | 2.85 | 14.43 | 16.51 | 43.28 | 2.79 | 14.14 | 16.48 |
| NH₂ | CF₃ | 4-methyl-3-pyridyl | 224-227 | 50.26 | 3.43 | 14.65 | 8.39 | 49.94 | 3.49 | 14.44 | 8.58 |
| | | | | | | | | | | | |
| NH₂ | CF₃ | 3-methyl-2-pyridyl | 235 | 50.26 | 3.43 | 14.65 | | 49.92 | 3.34 | 14.43 | |
| NH₂ | CF₃ | 2-thienyl | 225.5-226.5 | 45.04 | 2.70 | 11.25 | 17.18 | 44.92 | 2.62 | 11.09 | 17.48 |
| | | | | | | | | | | | |
| NH₂ | CF₃ | 2-quinolyl | 245 | 54.54 | 3.13 | 13.39 | | 54.35 | 2.92 | 13.38 | |
| | | | | | | | | | | | |
| NH₂ | CN | 5-methyl-3-pyridyl | 280-283 | 54.46 | 3.71 | 19.85 | | 54.84 | 3.83 | 19.50 | |

### Example 1

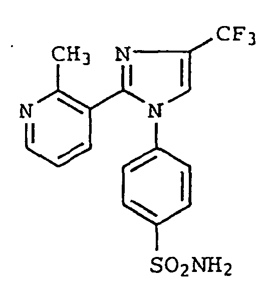

### 4-[2-(2-Methylpyridin-3-yl)-4-(trifluoromethyl)-1H-imidazol-1-yl]benzenesulfonamide

### Step 1: Preparation of 4-[(2,5-dimethyl-1H-pyrrol-1-yl)sulfonyl]nitrobenzene

A mixture of 4-nitrobenzenesulfonamide (30.3 g, 0.15 mol), acetonylacetone (34.2 g, 0.30 mol) and 4-toluenesulfonic acid (3.0 g, catalyst) in 200 mL of toluene was heated at reflux under nitrogen using a Dean-Stark trap for 18 hours. The reaction was cooled and filtered through silica gel (700 g), eluting with mixtures of ethyl acetate and hexane. Removal of solvent *in vacuo* gave a crude brown solid. The crude product in ethyl acetate was treated with activated charcoal, and recrystallized from ethyl acetate and hexane to afford 4-[(2,5-dimethyl-1H-pyrrol-1-yl)sulfonyl]nitrobenzene (32.5 g, 77%) as a light yellow solid: mp (DSC): 101-103 °C. Anal. Calc'd. for C₁₂H₁₂N₂O₄S: C, 51.42; H, 4.32; N, 9:99; S, 11.44. Found: C, 51.62; H, 4.18; N, 9.96; S, 11.31.

### Step 2: Preparation of 4-[(2,5-dimethyl-1H-pyrrol-1-yl)sulfonyl]benzenamine

A mixture of 4-[(2,5-dimethyl-1H-pyrrol-1-yl)sulfonyl]nitrobenzene (Step 1) (7.3 g, 26 mmol) and Raney Nickel (0.7 g) in 70 mL of methanol was hydrogenated in a Parr apparatus at a pressure of 50 psi. After 3 hours, the catalyst was filtered and the filtrate was concentrated to give 4-[(2,5-dimethyl-1H-pyrrol-1-yl)sulfonyl]benzenamine (6.4 g) as a pale yellow solid: mp (DSC): 110-111°C. Anal. Calc'd. for C₁₂H₁₄N₂O₂S: C, 57.58; H, 5.64; N, 11.16; S, 12.81. Found: C, 57.44; H, 5.78; N, 11.11; S, 12.30.

### Step 3: Preparation of 1-[4-[(2,5-dimethyl-1H-pyrrol-1-yl)sulfonyl]phenyl]-4,5-dihydro-2-(2-methylpyridin-3-yl)-4-(trifluoromethyl)-1H-imidazol-4-ol

To a solution of sodium bis(trimethylsilyl)amide (120 mL of 1.0 M in tetrahydrofuran, 0.12 mol) was added dropwise a solution of 4-[(2,5-dimethyl-1H-pyrrol-1-yl)sulfonyl]benzenamine (Step 2) (28.43 g, 0.114 mol) in 35 mL of tetrahydrofuran at room temperature. The dark solution was stirred for 10 minutes. A solution of 3-cyano-2-methylpyridine in 20 mL of tetrahydrofuran was added rapidly. The reaction mixture was stirred for 16 hours, poured into 1 L of water and extracted with ethyl acetate (500 mL). The organic layer was washed with brine, dried over magnesium sulfate and filtered. The filtrate was concentrated to give 22.0 g of crude amidine as a pale yellow solid which was used in next step without purification. To a suspension of the crude amidine (21.9 g, 0.065 mol) and sodium bicarbonate (8.20 g, 0.098 mol) in 600 mL of isopropanol at 50 °C, was added a solution of 3-bromo-1,1,1-trifluoroacetone (18.6 g, 0.098 mol) in 30 mL of isopropanol over 30 minutes. The mixture was stirred at 80 °C for 4 hours, cooled and filtered. The filtrate was concentrated and the residue was treated with ethyl acetate/hexane to give 28.6 g of 1-[4-[(2,5-dimethyl-1H-pyrrol-1-yl)sulfonyl]phenyl]-4,5-dihydro-2-(2-methylpyridin-3-yl)-4-(trifluoromethyl)-1H-imidazol-4-ol as a yellowish solid (53%): mp (DSC) 213-216°C. Anal. Calc'd. for C₂₂H₁₉F₃N₄O₃S: C, 55.46, H, 4.02, N, 11.76, S, 6.73. Found: C, 54.71, H, 4.40, N, 11.21, S, 6.78.

### Step 4: Preparation of 3-[1-[4-[(2,5-dimethyl-1H-pyrrol-1-yl)sulfonyl]phenyl]-4-(trifluoromethyl)-1H-imidazol-2-yl]-2-methylpyridine

A mixture of 1-[4-[(2,5-dimethyl-1H-pyrrol-1-yl)sulfonyl]phenyl]-4,5-dihydro-2-(2-methylpyridin-3-yl)-4-(trifluoromethyl)-1H-imidazol-4-ol (Step 3) (18.0 g, 38 mmol) and p-toluenesulfonic acid (1.8 g) in 400 mL of toluene was heated at reflux with a Dean-Stark trap under a nitrogen atmosphere for 36 hours. The mixture was cooled to room temperature and filtered. The filtrate was basified with ammonium hydroxide and extracted with ethyl acetate (400 mL). The organic layer was washed with brine, dried over magnesium sulfate and filtered. The filtrate was concentrated and purified by chromatography on silica gel (ethyl acetate/hexane, 95:5) to give 3-[1-[4-[2,5-dimethyl-1H-pyrrol-1-yl)sulfonyl]phenyl]-4-(trifluoromethyl)-1H-imidazol-2-yl]-2-methylpyridine as a white solid (11.86 g, 72%): mp (DSC): 141-143°C. Anal. Calc'd. for C₂₂H₁₉F₃N₄O₂S: C, 57.64; H, 3.74; N, 12.22; S, 6.99. Found: C, 57.27; H, 4.03; N, 11.79; S, 7.05.

### Step 5: Preparation of 4-[2-(2-methylpyridin-3-yl)-4-(trifluoromethyl)-1H-imidazol-1-yl]benzenesulfonamide

A mixture of 3-[1-[4-[(2,5-dimethyl-1H-pyrrol-1-yl)sulfonyl]phenyl]-4,5-dihydro-4-(trifluoromethyl)-1H-imidazol-2-yl]-2-methylpyridine (Step 4) (4.6 g, 0.01 mol) in 75 mL of TFA and 25 mL of water was heated at reflux for 2 hours. The solution was cooled, treated with 400 mL of water and basified with sodium bicarbonate to pH 8. The aqueous phase was extracted with ethyl acetate (400 mL). The organic layer was washed with brine, dried over magnesium sulfate and filtered. The filtrate was concentrated and the crude was purified by chromatography on silica gel (ethyl acetate/acetone, 95:5) to afford 4-[2-(2-methylpyridin-3-yl)-4-(trifluoromethyl)-1H-imidazol-1-yl]benzenesulfonamide as a white solid (3.0 g, 78%): mp (DSC): 235-237°C. Anal. Calc'd. for C₁₆H₁₃F₃N₃O₂S: C, 50.26; H, 3.43; N, 14.65; S, 8.39. Found: C, 50.06; H,3.29; N, 14.4; S, 8.52.

Although this invention has been described with respect to specific embodiments, the details of these embodiments are not to be construed as limitations.

## Claims

1. A process of making a compound of the formula 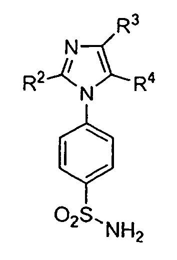 or a pharmaceutically-acceptable salt thereof, said method comprising
a) protecting the sulfonamide group of 4-nitrobenzenesulfonamide with acetonylacetone and toluenesulfonic acid to form a sulfonyl-pyrrole of the formula 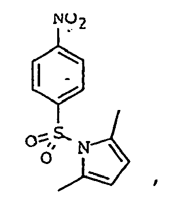
b) reducing the nitro group to form a compound of the formula 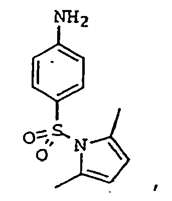
c) treating the amine with a nitrile in the presence of a base to form an amidine of the formula 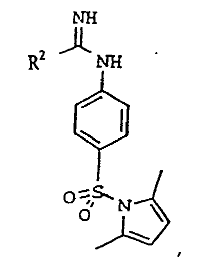
d) treating the amidine with a compound of the formula 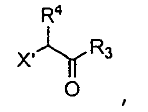 in the present of a base to form an imidazolyl derivative of the formula 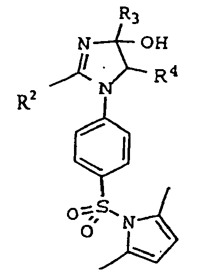
e) dehydrating the imidazolyl derivative to form a compound of the formula 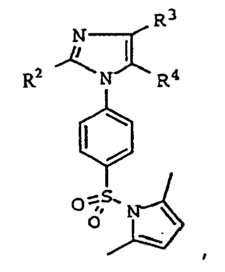
f) deprotecting the sulfonyl-pyrrole to form a compound of the formula 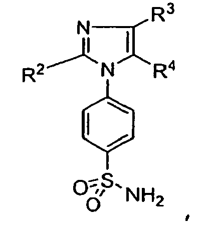 wherein
R² is selected from imidazolyl, thienyl, thiazolyl, pyrrolyl, oxazolyl, isoxazolyl, triazolyl, pyrimidinyl, isoquinolyl, quinolinyl, indolyl, benzimidazolyl, pyrazolyl and pyridyl, wherein R² is optionally substituted at a substitutable position with one or more radicals independently selected from methylthio, methyl, ethyl, isopropyl, *tert*-butyl, isobutyl, pentyl, hexyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methoxy, methylenedioxy, ethoxy, propoxy, n-butoxy, hydroxymethyl, hydroxyethyl, methoxymethyl, ethoxymethyl, and trifluoromethoxy;
R³ is a radical selected from hydrido, methyl, ethyl, isopropyl, *tert*-butyl, isobutyl, pentyl, hexyl, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, and 2-methylphenylthiomethyl;
R⁴ is a radical selected from hydrido, C₁-C₆ alkyl, and halogen; and
X' is chloro or bromo.

2. A method according to claim 1, wherein R⁴ is hydrido.

3. A method according to Claim 1 wherein the compound is selected from the group consisting of
4-[2-(6-methylpyrindin-2-yl)-4-trifluoromethyl-1H-imidazol-1-yl]benzenesulfonamide;
4-[2-(2-methylthiazol-4-yl)-4-trifluoromethyl-1H-imidazol-1-yl]benzenesulfonamide;
4-[2-(4-methylpyrindin-3-yl)-4-trifluoromethyl-1H-imidazol-1-yl]benzenesulfonamide;
4-[2-(3-methylpyrindin-2-yl)-4-trifluoromethyl-1H-imidazol-1-yl]benzenesulfonamide;
4-[2-(2-thienyl)-4-trifluoromethyl-1H-imidazol-1-yl]benzenesulfonamide;
4-[4-cyano-2-(5-methylpyrindin-3-yl)-1H-imidazol-1-yl]benzenesulfonamide;
4-[2-(2-quinolinyl)-4-trifluoromethyl-1H-imidazol-1-yl]benzenesulfonamide;
4-[2-(3-methoxypyridin-5-yl)-4-(trifluoromethyl)-1H-imidazol-1-yl]benzenesulfonamide;
4-[2-(4-methylpyridin-2-yl)-4-(trifluoromethyl)-1H-imidazol-1-yl]benzenesulfonamide;
4-[2-(5-methylpyridin-3-yl)-4-(trifluoromethyl)-1H-imidazol-1-yl]benzenesulfonamide;
4-[2-(2-methylpyridin-3-yl)-4-(trifluoromethyl)-1H-imidazol-1-yl]benzenesulfonamide;
4-[2-(5-methylpyridin-2-yl)-4-(trifluoromethyl)-1H-imidazol-1-yl]benzenesulfonamide;
4-[2-(4-methylpyridin-2-yl)-4-(trifluoromethyl)-1H-imidazol-1-yl]benzenesulfonamide;
4-[2-(6-methoxypyridin-2-yl)-4-(trifluoromethyl)-1H-imidazol-1-yl]benzenesulfonamide;
4- [2- (5-methoxypyridin-2-yl) -4- (trifluoromethyl) -1H-imidazol-1-yl]benzenesulfonamide;
4-[2-(4-methoxypyridin-2-yl)-4-(trifluoromethyl)-1H-imidazol-1-yl]benzenesulfonamide;
4-[2-(5-methoxypyridin-3-yl)-4-(trifluoromethyl)-1H-imidazol-1-yl]benzenesulfonamide;
4-[4-methyl-2-(3-pyridinyl)-1H-imidazol-1-yl]benzenesulfonamide;
9-[2-(pyridin-3-yl)-4-(trifluoromethyl)-1H-imidazol-1-yl]benzenesulfonamide;
4-[2-[6-(methylthio)pyridin-3-yl]-4-trifluoromethyl)-1H-imidazol-1-yl]benzenesulfonamide;
4-[4-(difluoromethyl)-2-(3-pyridinyl)-1H-imidazol-1-yl]benzenesulfonamide;
4-[2-(6-methylpyridin-3-yl)-4-(trifluoromethyl)-1H-imidazol-1-yl]benzenesulfonamide; and
pharmaceutically acceptable salts thereof.

4. A method according to claim 1 where the compound is selected from the group consisting of
4-[2-(pyrindin-3-yl)-4-[[(methylphenyl)thio]methyl]-1H-imidazol-1-yl]benzenesulfonamide;
4-[2-(6-methylpyrindin-2-yl)-4-trifluoromethyl-1H-imidazol-1-yl]benzenesulfonamide;
4-[2-(4-methylpyrindin-3-yl)-4-trifluoromethyl-1H-imidazol-1-yl]benzenesulfonamide;
4-[2-(3-methylpyrindin-2-yl)-4-trifluoromethyl-1H-imidazol-1-yl]benzenesulfonamide;
4-[2-(2-thienyl)-4-trifluoromethyl-1H-imidazol-1-yl]benzenesulfonamide;
4-[4-difluoromethyl-2-(pyrindin-3-yl)-1H-imidazol-1-yl]benzenesulfonamide;
4-[4-cyano-2-(pyrindin-3-yl)-1H-imidazol-1-yl]benzenesulfonamide;
4-[4-cyano-2-(5-methylpyrindin-3-yl)-1H-imidazol-1-yl]benzenesulfonamide;
4-[2-(2-quinolinyl)-4-trifluoromethyl-1H-imidazol-1-yl]benzenesulfonamide;
4-[2-(1-methyl-1H-pyrazol-4-yl)-4-trifluoromethyl-1H-imidazol-1-yl]benzenesulfonamide;
4-[2-(1-methyl-1H-imidazol-4-yl)-4-trifluoromethyl-1H-imidazol-1-yl]benzenesulfonamide;
4-[2-(1-methyl-1H-imidazol-5-yl)-4-trifluoromethyl-1H-imidazol-1-yl]benzenesulfonamide;
4-[2-(1-methyl-1H-imidazol-2-yl)-4-trifluoromethyl-1H-imidazol-1-yl]ben2enesulfonamide;
4-[2-(4-methylthiazol-2-yl)-4-trifluoromethyl-1H-imidazol-1-yl]benzenesulfonamide;
4-[2-(2-methylthiazol-5-yl)-4-trifluoromethyl-1H-imidazol-1-yl]benzenesulfonamide;
4-[2-(5-methylisoxazol-3-yl)-4-trifluoromethyl-1H-imidazol-1-yl]benzenesulfonamide;
4-[2-(5-pyrimidinyl)-4-trifluoromethyl-1H-imidazol-1-yl]benzenesulfonamide;
4-[2-(pyrazin-2-yl)-4-(trifluoromethyl)-1H-imidazol-1-yl]benzenesulfonamide;
4-[2-(quinol-3-yl)-4-(trifluoromethyl)-1H-imidazol-1-yl]benzenesulfonamide; and
pharmaceutically acceptable salts thereof.

5. A method according to any one of claims 1-4, wherein the protection of the sulfonamide group is performed in toluene.

6. A method according to any one of claims 1-5, wherein the nitro group is reduced by catalytic hydrogenation.

7. A method according to claim 6, wherein Raney-Nickel is the catalyst.

8. A method according to any one of claims 1-7, wherein the base is selected from the group consisting of sodium bis(trimethylsilyl)amide, sodium hydride, sodium methoxide, n-butyllithium and lithium diisopropylamide.

9. A method according to any one of claims 1-8, wherein the reaction of the amine with the nitrile is performed in the presence of a solvent, which is selected from the group consisting of dimethylsulfoxide, tetrahydrofuran, dimethoxyethane, and methanol.

10. A method according to any one of claims 1-9, wherein the base in step d is selected from the group consisting of sodium bicarbonate, potassium carbonate, sodium carbonate, potassium bicarbonate and *N,N'-*diisopropylethylamine.

11. A method according to claim 10, wherein step d further comprises the presence of a solvent, which is selected from the group consisting of isopropanol, acetone and dimethylformamide.

12. A method according to any one of claims 1-11, wherein the dehydration is performed with an acid.

13. A method according to claim 12, wherein the acid is 4-toluenesulfonic acid.

14. A method according to claim 12, wherein the dehydration is carried out in a solvent, that is selected from the group consisting of toluene, xylene and benzene.

15. A method according to any one of claims 1-14, wherein the deprotection of the sulfonyl-pyrrole is done in aqueous trifluoroacetic acid.

16. A method according to any one of claims 1-15, wherein the deprotection is performed in a refluxing solvent.

## Patentansprüche

1. Verfahren zur Herstellung einer Verbindung der Formel 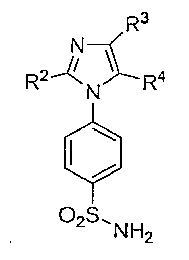 oder eines pharmazeutisch annehmbaren Salzes davon, wobei das Verfahren umfasst
a) Schützen der Sulfonamidgruppe von 4-Nitrobenzolsulfonamid mit Acetonylaceton und Toluolsulfonsäure zur Herstellung eines Sulfonyl-Pyrrols der Formel 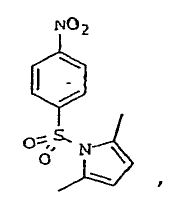
b) Reduktion der Nitrogruppe zur Herstellung einer Verbindung der Formel 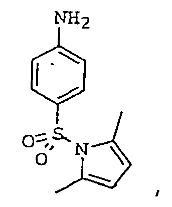
c) Behandeln des Amins mit einem Nitril in Gegenwart einer Base zur Herstellung eines Amidins der Formel 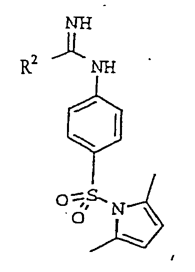
d) Behandeln des Amidins mit einer Verbindung der Formel 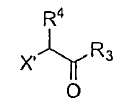 in Gegenwart einer Base zur Herstellung eines Imidazolylderivats der Formel 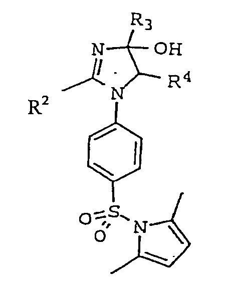
e) Dehydratisieren des Imidazolylderivats zur Herstellung einer Verbindung der Formel 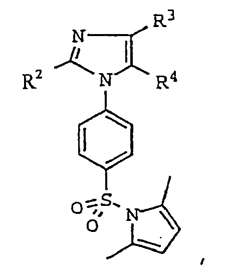
f) Entschützen des Sulfonyl-Pyrrols zur Herstellung einer Verbindung der Formel 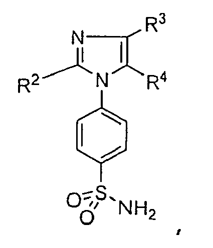
worin
R² ausgewählt ist aus Imidazolyl, Thienyl, Thiazolyl, Pyrrolyl, Oxazolyl, Isoxazolyl, Triazolyl, Pyrimidinyl, Isochinolyl, Chinolyl, Indolyl, Benzimidazolyl, Pyrazolyl und Pyridyl, worin R² gegebenenfalls an einer substituierbaren Position substituiert ist mit einem oder mehreren Resten, die unabhängig voneinander ausgewählt sind aus Methylthio, Methyl, Ethyl, Isopropyl, *tert.*-Butyl, Isobutyl, Pentyl, Hexyl, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Methoxy, Methylendioxy, Ethoxy, Propoxy, n-Butoxy, Hydroxymethyl, Hydroxyethyl, Methoxymethyl, Ethoxymethyl und Trifluormethoxy;
R³ ein Rest ist, der ausgewählt ist aus Hydrido, Methyl, Ethyl, Isopropyl, *tert*-Butyl, Isobutyl, Pentyl, Hexyl, Cyano, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl und 2-Methylphenylthiomethyl;
R⁴ ein Rest ist, der ausgewählt ist aus Hydrido, C₁-C₆-Alkyl und Halogen und
X' Chlor oder Brom ist.

2. Verfahren nach Anspruch 1, worin R⁴ Hydrido ist.

3. Verfahren nach Anspruch 1, worin die Verbindung ausgewählt ist aus der Gruppe, bestehend aus
4-[2-(6-Methylpyridin-2-yl)-4-trifluormethyl-1H-imidazol-1-yl]benzolsulfonamid;
4-[2-(2-Methylthiazol-4-yl)-4-trifluormethyl-1H-imidazol-1-yl]benzolsulfonamid;
4-[2-(4-Methylpyridin-3-yl)-4-trifluormethyl-1H-imidazol-1-yl]benzolsulfonamid;
4-[2-(3-Methylpyridin-2-yl)-4-trifluormethyl-1H-imidazol-1-yl]benzolsulfonamid;
4-[2-(2-Thienyl)-4-trifluormethyl-1H-imidazol-1-yl]benzolsulfonamid;
4-[4-Cyano-2-(5-methylpyridin-3-yl)-1H-imidazol-1-yl]benzolsulfonamid;
4-[2-(2-Chinolinyl)-4-trifluormethyl-1H-imidazol-1-yl]benzolsulfonamid;
4-[2-(3-Methoxypyridin-5-yl)-4-(trifluormethyl)-1H-imidazol-1-yl]benzolsulfonamid;
4-[2-(4-Methylpyridin-2-yl)-4-(trifluormethyl)-1H-imidazol-1-yl]benzolsulfonamid;
4-[2-(5-Methylpyridin-3-yl)-4-(trifluormethyl)-1H-imidazol-1-yl]benzolsulfonamid;
4-[2-(2-Methylpyridin-3-yl)-4-(trifluormethyl)-1H-imidazol-1-yl]benzolsulfonamid;
4-[2-(5-Methylpyridin-2-yl)-4-(trifluormethyl)-1H-imidazol-1-yl]benzolsulfonamid;
4-[2-(4-Methylpyridin-2-yl)-4-(trifluormethyl)-1H-imidazol-1-yl]benzolsulfonamid;
4-[2-(6-Methoxypyridin-2-yl)-4-(trifluormethyl)-1H-imidazol-1-yl]benzölsulfonamid;
4-[2-(5-Methoxypyridin-2-yl)-4-(trifluormethyl)-1H-imidazol-1-yl]benzolsulfonamid;
4-[2-(4-Methoxypyridin-2-yl)-4-(trifluormethyl)-1H-imidazol-1-yl]benzolsulfonamid;
4-[2-(5-Methoxypyridin-3-yl)-4-(trifluormethyl)-1H-imidazol-1-yl]benzolsulfonamid;
4-[4-Methyl-2-(3-pyridinyl)-1H-imidazol-1-yl]benzolsulfonamid;
4-[2-(Pyridin-3-yl)-4-(trifluormethyl)-1H-imidazol-1-yl]benzolsulfonamid;
4-[2-[6-(Methylthio)pyridin-3-yl]-4-(trifluormethyl)-1H-imidazol-1-yl]benzolsulfonamid;
4-[4-(Difluormethyl)-2-(3-pyridinyl)-1H-imidazol-1-yl]benzolsulfonamid;
4-[2-(6-Methylpyridin-3-yl)-4-(trifluormethyl)-1H-imidazol-1-yl]benzolsulfonamid und
pharmazeutisch annehmbaren Salzen davon.

4. Verfahren nach Anspruch 1, worin die Verbindung ausgewählt ist aus der Gruppe, bestehend aus
4-[2-(Pyridin-3-yl)-4-[[(methylphenyl)thio]methyl]-1H-imidazol-1-yl]benzolsulfonamid;
4-[2-(6-Methylpyridin-2-yl)-4-trifluormethyl-1H-imidazol-1-yl]benzolsulfonamid;
4-[2-(4-Methylpyridin-3-yl)-4-trifluormethyl-1H-imidazol-1-yl]benzolsulfonamid;
4-[2-(3-Methylpyridin-2-yl)-4-trifluormethyl-1H-imidazol-1-yl]benzolsulfonamid;
4-[2-(2-Thienyl)-4-trifluormethyl-1H-imidazol-1-yl]benzolsulfonamid;
4-[4-Difluormethyl-2-(pyridin-3-yl)-1H-imidazol-1-yl]benzolsulfonamid;
4-[4-Cyano-2-(pyridin-3-yl)-1H-imidazol-1-yl]benzolsulfonamid;
4-[4-Cyano-2-(5-methylpyridin-3-yl)-1H-imidazol-1-yl]benzolsulfonamid;
4-[2-(2-Chinolinyl)-4-trifluormethyl-1H-imidazol-1-yl]benzolsulfonamid;
4-[2-(1-Methyl-1H-pyrazol-4-yl)-4-trifluormethyl-1H-imidazol-1-yl]benzolsulfonamid;
4-[2-(1-Methyl-1H-imidazol-4-yl)-4-trifluormethyl-1H-imidazol-1-yl]benzolsulfonamid;
4-[2-(1-methyl-1H-imidazol-5-yl)-4-trifluormethyl-1H-imidazol-1-yl]benzolsulfonamid;
4-[2-(1-Methyl-1H-imidazol-2-yl)-4-trifluormethyl-1H-imidazol-1-yl]benzolsulfonamid;
4-[2-(4-Methylthiazol-2-yl)-4-trifluormethyl-1H-imidazol-1-yl]benzolsulfonamid;
4-[2-(2-Methylthiazol-5-yl)-4-trifluormethyl-1H-imidazol-1-yl]benzolsulfonamid;
4-[2-(5-Methylisoxazol-3-yl)-4-trifluormethyl-1H-imidazol-1-yl]benzolsulfonamid;
4-[2-(5-Pyrimidinyl)-4-trifluormethyl-1H-imidazol-1-yl]benzolsulfonamid;
4-[2-(Pyrazin-2-yl)-4-(trifluormethyl)-1H-imidazol-1-yl]benzolsulfonamid;
4-[2-(Chinol-3-yl)-4-(trifluormethyl)-1H-imidazol-1-yl]benzolsulfonamid und
pharmazeutisch annehmbaren Salzen davon.

5. Verfahren nach einem der Ansprüche 1-4, worin das Schützen der Sulfonamidgruppe in Toluol durchgeführt wird.

6. Verfahren nach einem der Ansprüche 1-5, worin die Nitrogruppe durch katalytische Hydrierung reduziert wird.

7. Verfahren nach Anspruch 6, worin Raney-Nickel der Katalysator ist.

8. Verfahren nach einem der Ansprüche 1-7, worin die Base ausgewählt ist aus der Gruppe, bestehend aus Natrium-bis(trimethylsilyl)amid, Natriumhydrid, Natriummethoxid, n-Butyllithium und Lithiumdiisopropylamid.

9. Verfahren nach einem der Ansprüche 1-8, worin die Reaktion des Amins mit dem Nitril in Gegenwart eines Lösungsmittels durchgeführt wird, das aus der Gruppe, bestehend aus Dimethylsulfoxid, Tetrahydrofuran, Dimethoxyethan und Methanol ausgewählt ist.

10. Verfahren nach einem der Ansprüche 1-9, worin die Base in Stufe d aus der Gruppe, bestehend aus Natriumbicarbonat, Kaliumcarbonat, Natriumcarbonat, Kaliumbicarbonat und N,N'-Diisopropylethylamin ausgewählt ist.

11. Verfahren nach Anspruch 10, worin Stufe d weiterhin die Gegenwart eines Lösungsmittels umfasst, das aus der Gruppe, bestehend aus Isopropanol, Aceton und Dimethylformamid ausgewählt ist.

12. Verfahren nach einem der Ansprüche 1-11, worin die Dehydration mit einer Säure durchgeführt wird.

13. Verfahren nach Anspruch 12, worin die Säure 4-Toluolsulfonsäure ist.

14. Verfahren nach Anspruch 12, worin die Dehydration in einem Lösungsmittel durchgeführt wird, das aus der Gruppe, bestehend aus Toluol, Xylol und Benzol ausgewählt ist.

15. Verfahren nach einem der Ansprüche 1-14, worin das Entschützen des Sulfonyl-Pyrrols in wässriger Trifluoressigsäure durchgeführt wird.

16. Verfahren nach einem der Ansprüche 1-15, worin das Entschützen in einem refluxierenden Lösungsmittel durchgeführt wird.

## Revendications

1. Procédé de production d'un composé de formule 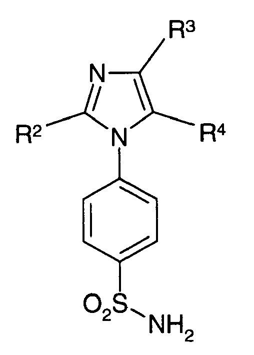 ou d'un sel pharmaceutiquement acceptable de celui-ci, ledit procédé comprenant
a) la protection du groupe sulfonamide du 4-nitrobenzènesulfonamide avec l'acétonylacétone et l'acide toluènesulfonique pour former un sulfonyl-pyrrole de formule 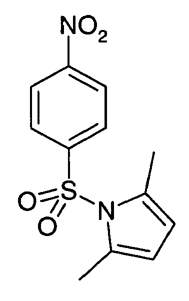
b) la réduction du groupe nitro pour former un composé de formule 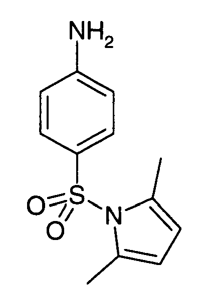
c) le traitement de l'amine avec un nitrile en présence d'une base pour former une amidine de formule 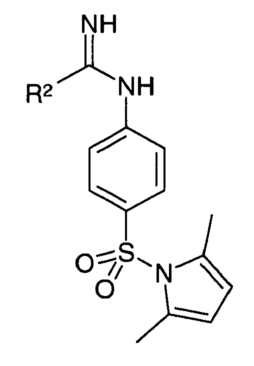
d) le traitement de l'amidine avec un composé de formule 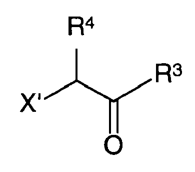 en présence d'une base pour former un dérivé imidazolyle de formule 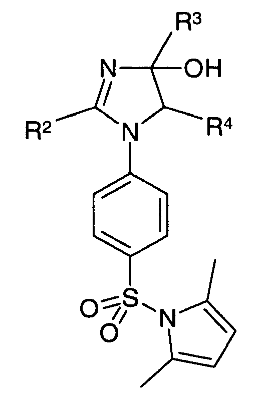
e) la déshydratation du dérivé imidazolyle pour former un composé de formule 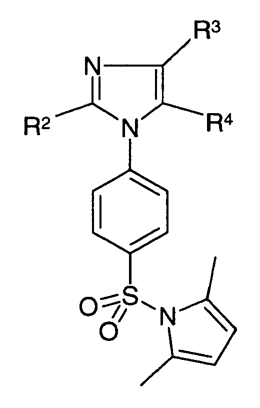
f) la déprotection du sulfonyl-pyrrole pour former un composé de formule 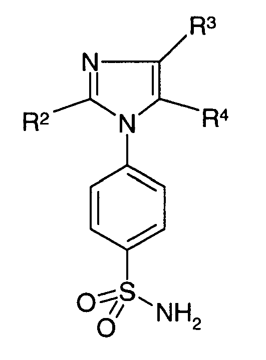
où
R² est choisi parmi imidazolyle, thiényle, thiazolyle, pyrrolyle, oxazolyle, isoxazolyle, triazolyle, pyrimidinyle, isoquinolyle, quinolinyle, indolyle, benzimidazolyle, pyrazolyle et pyridyle, où R² est éventuellement substitué à une position substituable avec un ou plusieurs radicaux choisis indépendamment parmi méthylthio, méthyle, éthyle, isopropyle, tert-butyle, isobutyle, pentyle, hexyle, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, méthoxy, méthylènedioxy, éthoxy, propoxy, n-butoxy, hydroxyméthyle, hydroxyéthyle, méthoxyméthyle, éthoxyméthyle et trifluorométhoxy ;
R³ est un radical choisi parmi hydruro, méthyle, éthyle, isopropyle, tert-butyle, isobutyle, pentyle, hexyle, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle et 2-méthylphénylthiométhyle ;
R⁴ est un radical choisi parmi hydruro, C₁-C₆ alkyle et halogène ; et
X' est chloro ou bromo.

2. Procédé selon la revendication 1 où R⁴ est hydruro.

3. Procédé selon la revendication 1 où le composé est choisi dans le groupe consistant en
4-[2-(6-méthylpyridin-2-yl)-4-trifluorométhyl-1H-imidazol-1-yl]benzènesulfonamide ;
4-[2-(2-méthylthiazol-4-yl)-4-trifluorométhyl-1H-imidazol-1-yl]benzènesulfonamide;
4-[2-(4-méthylpyridin-3-yl)-4-trifluorométhyl-1H-imidazol-1-yl]benzènesulfonamide;
4-[2-(3-méthylpyridin-2-yl)-4-trifluorométhyl-1H-imidazol-1-yl]benzènesulfonamide ;
4-[2-(2-thiényl)-4-trifluorométhyl-1H-imidazol-1-yl]benzènesulfonamide ;
4-[4-cyano-2-(5-méthylpyridin-3-yl)-1H-imidazol-1-yl]benzènesulfonamide ;
4-[2-(2-quinolinyl)-4-trifluorométhyl-1H-imidazol-1-yl]benzènesulfonamide ;
4-[2-(3-méthoxypyridin-5-yl)-4-(trifluorométhyl)-1H-imidazol-1-yl]-benzènesulfonamide ;
4-[2-(4-méthylpyridin-2-yl)-4-(trifluorométhyl)-1H-imidazol-1-yl]benzènesulfonamide ;
4-[2-(5-méthylpyridin-3-yl)-4-(trifluorométhyl)-1H-imidazol-1-yl]benzènesulfonamide ;
4-[2-(2-méthylpyridin-3-yl)-4-(trifluorométhyl)-1H-imidazol-1-yl]benzènesulfonamide ;
4-[2-(5-méthylpyridin-2-yl)-4-(trifluorométhyl)-1H-imidazol-1-yl]benzènesulfonamide ;
4-[2-(4-méthylpyridin-2-yl)-4-(trifluorométhyl)-1H-imidazol-1-yl]benzènesulfonamide ;
4-[2-(6-méthoxypyridin-2-yl)-4-(trifluorométhyl)-1H-imidazol-1-yl]benzènesulfonamide ;
4-[2-(5-méthoxypyridin-2-yl)-4-(trifluorométhyl)-1H-imidazol-1-yl]-benzènesulfonamide ;
4-[2-(4-méthoxypyridin-2-yl)-4-(trifluorométhyl)-1H-imidazol-1-yl]-benzènesulfonamide ;
4-[2-(5-méthoxypyridin-3-yl)-4-(trifluorométhyl)-1H-imidazol-1-yl]-benzènesulfonamide ;
4-[4-méthyl-2-(3-pyridinyl)-1H-imidazol-1-yl]benzènesulfonamide ;
4-[2-(pyridin-3-yl)-4-(trifluorométhyl)-1H-imidazol-1-yl]benzènesulfonamide ;
4-[2-[6-(méthylthio)pyridin-3-yl]-4-trifluorométhyl)-1H-imidazol-1-yl]-benzènesulfonamide ;
4-[4-(difluorométhyl)-2-(3-pyridinyl)-1H-imidazol-1-yl]benzènesulfonamide ;
4-[2-(6-méthylpyridin-3-yl)-4-(trifluorométhyl)-1H-imidazol-1-yl]benzènesulfonamide ; et
les sels pharmaceutiquement acceptables de ceux-ci.

4. Procédé selon la revendication 1 où le composé est choisi dans le groupe consistant en
4-[2-(pyridin-3-yl)-4-[[(méthylphényl)thio]méthyl]-1H-imidazol-1-yl]-benzènesulfonamide ;
4-[2-(6-méthylpyridin-2-yl)-4-trifluorométhyl-1H-imidazol-1-yl]benzènesulfonamide ;
4-[2-(4-méthylpyridin-3-yl)-4-trifluorométhyl-1H-imidazol-1-yl]benzènesulfonamide ;
4-[2-(3-méthylpyridin-2-yl)-4-trifluorométhyl-1H-imidazol-1-yl]benzènesulfonamide ;
4-[2-(2-thiényl)-4-trifluorométhyl-1H-imidazol-1-yl]-benzènesulfonamide;
4-[4-difluorométhyl-2-(pyridin-3-yl)-1H-imidazol-1-yl]benzènesulfonamide ;
4-[4-cyano-2-(pyridin-3-yl)-1H-imidazol-1-yl]benzène-sulfonamide ;
4-[4-cyano-2-(5-méthylpyridin-3-yl)-1H-imidazol-1-yl]benzènesulfonamide ;
4-[2-(2-quinolinyl)-4-trifluorométhyl-1H-imidazol-1-yl]benzènesulfonamide ;
4-[2-(1-méthyl-1H-pyrazol-4-yl)-4-trifluorométhyl-1H-imidazol-1-yl]-benzènesulfonamide ;
4-[2-(1-méthyl-1H-imidazol-4-yl)-4-trifluorométhyl-1H-imidazol-1-yl]-benzènesulfonamide ;
4-[2-(1-méthyl-1H-imidazol-5-yl)-4-trifluorométhyl-1H-imidazol-1-yl]-benzènesulfonamide ;
4-[2-(1-méthyl-1H-imidazol-2-yl)-4-trifluorométhyl-1H-imidazol-1-yl]-benzènesulfonamide ;
4-[2-(4-méthylthiazol-2-yl)-4-trifluorométhyl-1H-imidazol-1-yl]benzènesulfonamide ;
4-[2-(2-méthylthiazol-5-yl)-4-trifluorométhyl-1H-imidazol-1-yl]benzènesulfonamide ;
4-[2-(5-méthylisoxazol-3-yl)-4-trifluorométhyl-1H-imidazol-1-yl]benzènesulfonamide ;
4-[2-(5-pyrimidinyl)-4-trifluorométhyl-1H-imidazol-1-yl]benzènesulfonamide ;
4-[2-(pyrazin-2-yl)-4-(trifluorométhyl)-1H-imidazol-1-yl]benzènesulfonamide ;
4-[2-(quinol-3-yl)-4-(trifluorométhyl)-1H-imidazol-1-yl]benzènesulfonamide ; et
les sels pharmaceutiquement acceptables de ceux-ci.

5. Procédé selon l'une quelconque des revendications 1-4 où la protection du groupe sulfonamide est accomplie dans le toluène.

6. Procédé selon l'une quelconque des revendications 1-5 où le groupe nitro est réduit par hydrogénation catalytique.

7. Procédé selon la revendication 6 où le nickel de Raney est le catalyseur.

8. Procédé selon l'une quelconque des revendications 1-7 où la base est choisie dans le groupe consistant en le bis(triméthylsilyl)amidure de sodium, l'hydrure de sodium, le méthylate de sodium, le n-butyllithium et le diisopropylamidure de lithium.

9. Procédé selon l'une quelconque des revendications 1-8 où la réaction de l'amine avec le nitrile est conduite en présence d'un solvant qui est choisi dans le groupe consistant en le diméthylsulfoxyde, le tétrahydrofurane, le diméthoxyéthane et le méthanol.

10. Procédé selon l'une quelconque des revendications 1-9 où la base dans l'étape d est choisie dans le groupe consistant en le bicarbonate de sodium, le carbonate de potassium, le carbonate de sodium, le bicarbonate de potassium et la *N,N'*-diisopropyléthylamine.

11. Procédé selon la revendication 10 où l'étape d comprend en outre la présence d'un solvant qui est choisi dans le groupe consistant en l'isopropanol, l'acétone et le diméthylformamide.

12. Procédé selon l'une quelconque des revendications 1-11 où la déshydratation est accomplie avec un acide.

13. Procédé selon la revendication 12 où l'acide est l'acide 4-toluènesulfonique.

14. Procédé selon la revendication 12 où la déshydratation est conduite dans un solvant qui est choisi dans le groupe consistant en le toluène, le xylène et le benzène.

15. Procédé selon l'une quelconque des revendications 1-14 où la déprotection du sulfonyl-pyrrole est accomplie dans l'acide trifluoroacétique aqueux.

16. Procédé selon l'une quelconque des revendications 1-15 où la déprotection est accomplie dans un solvant à reflux.
